# EUROPEAN PATENT APPLICATION

(11) **EP 2 191 848 A1**
(43) Date of publication of application: **02.06.2010**
(21) Application number: 08828416.1
(22) Date of filing: 28.08.2008
(51) Int. Cl.: A61K 47/38, A61K 9/22, A61K 31/155, A61K 31/439, A61K 47/10, A61K 47/12, A61K 47/32, A61K 47/34, A61K 47/36

(54) **SUSTAINED RELEASE PREPARATION AND PROCESS FOR PRODUCTION THEREOF**

(30) Priority: 31.08.2007 JP 2007226491
(71) Applicant: Daiichi Sankyo Company, Limited, Chuo-ku Tokyo 103-8426 (JP)
(72) Inventor: KANAMARU, Taro, Shinagawa-ku Tokyo 140-8710 (JP); TAJIRI, Shinichiro, Shinagawa-ku Tokyo 140-8710 (JP)
(74) Representative: Fairbairn, Angus Chisholm
(86) International application number: PCT/JP2008/065385
(87) International publication number: WO 2009/028598

(57) **Abstract**

The present invention provides a preparation which is a sustained-release preparation capable of providing a dissolution profile that permits once-daily administration even in the case of a highly water-soluble drug, and which can exhibit stable drug dissolution behavior and can be reduced in its size; and a method for producing the same. A preparation containing a highly water-soluble drug, a gel-forming polymer, and a low-melting lipophilic substance is usable as a sustained-release preparation that permits once-daily administration, and enables reduction in its size and simple production thereof, and thus, the objects have been achieved.

## Description

### Technical Field

The present invention relates to a preparation which can stably control drug dissolution without being affected by variation in the dissolution conditions and which has a size that does not cause difficulty in taking the preparation; and to a method for producing the same.

### Background Art

Controlled-release oral preparations have the advantage of reducing burdens on patients by decreasing dose frequency and the like and improving low compliance. As a technique for such a controlled-release oral preparation, means for maintaining blood concentration by releasing a drug in a sustained manner, i.e., so-called sustained-release preparations are widely known.

As sustained-release preparations, according to differences in controlled drug release mechanism, there are matrix type preparations in which a drug and a sustained-release agent are uniformly mixed; and coated type preparations in which a drug layer containing a drug is coated with a sustained-release agent. The matrix type preparation is advantageous in terms of practical use because the structure and production process thereof are simpler than those of the coated type preparation.

However, in the case where the dissolution rate is to be controlled for a long period of time with the use of the matrix type preparation, when the solubility of a drug is extremely high, or when a large amount of a drug needs to be comprised in the preparation, a large amount of a sustained-release agent and the like needs to be added thereto, and therefore, there is a concern that the size of the preparation is increased. Further, as the sustained-release agent to be used in the matrix type preparation, a gel-forming polymer typified by hydroxypropyl cellulose (HPC) or hypromellose (hydroxypropylmethyl cellulose (HPMC)) is widely used. However, such a gel-forming polymer powder is known to have a low fluidity at the time of tableting, and there is a problem that this tendency becomes more pronounced when the gel-forming polymer is in the form of a finer powder which exhibits a strong sustained-release effect.

As a method of improving the flowability of a gel-forming polymer, a method of wet granulation of a gel-forming polymer is known (Patent Document 1). However, this method has a problem that when a large amount of a gel-forming polymer is added in order to ensure sustained release, the gel-forming polymer aggregates due to a binder solution used in production or adheres to production apparatus, which makes production difficult.

Further, as a granulation method other than wet granulation, melt granulation can be exemplified. As a method for producing a preparation by melt granulation in the case where a gel-forming polymer is used as a sustained-release agent, a method for producing a preparation by adding polyethylene glycol (PEG) to a gel-forming polymer, followed by melt granulation (Patent Document 2) and a preparation obtained by melt granulation of a hydrophilic cellulose, PEG, and a glyceryl ester as disclosed in Patent Document 3 have been reported. However, there is a concern that a preparation produced by such a method does not have sufficient strength and cannot sufficiently withstand mechanical stress (shear) in the gastrointestinal tract. Further, a method using a lipid alone as a low-melting substance in melt granulation has been attempted in Non-Patent Document 1. However, in the formulation disclosed in Non-Patent Document 1, since low-substituted HPC (L-HPC) does not function as a gel-forming polymer because it is insoluble in water, the content of HPC which functions as a gel-forming polymer is about 5% or less of the total amount, which is insufficient to ensure sustained release for permitting once-daily administration.

Further, as a method of avoiding the effect of mechanical strength in the gastrointestinal tract on a matrix type preparation, a method of adding a disintegration suppression substance such as an ethyl acrylate-methyl methacrylate-trimethylammonium ethyl methacrylate chloride copolymer (trade name: Eudragit RS and RL) or ethyl cellulose to an outer shell portion of a press-coated tablet (Patent Document 4) has been reported. However, since permeation of water into the tablet is restricted, this method has problems in that drug dissolution is difficult in the lower gastrointestinal tract where water is scarce, the size of the tablet is increased, the production process becomes complicated, etc.

Further, as a preparation containing a drug having a high water solubility and a method for producing the same, a technique in which cevimeline hydrochloride is coated with an insoluble polymer or the like, followed by mixing with a gel-forming polymer is known (Patent Document 5). However, in this preparation, the drug having a high water solubility needs to be coated in the production process, and therefore, there are problems in that the number of processes is large, the production method is complicated, the production cost is high, and the size of the tablet is increased.
Patent Document 1: JP-A-2000-34224
Patent Document 2: U.S. Patent Application Publication No. 2002/0160050
Patent Document 3: U.S. Patent No. 5403593
Patent Document 4: U.S. Patent No. 5861173
Patent Document 5: U.S. Patent No. 6419954
Non-Patent Document 1: "S.T.P. Pharma Sciences" 11(2), pp. 145-150, 2000

### Disclosure of the Invention

### Problems that the Invention is to Solve

An object of the invention is to provide a preparation which is a sustained-release preparation capable of providing a dissolution profile that permits once-daily administration even in the case of a water-soluble drug, **characterized in that:**
the size of the preparation is made as small as possible so as to increase the ease of taking the preparation;
the production method thereof is not complicated;
the fluidity of a sustained-release agent powder (or granulated product) at the time of tableting is favorable; and
the dissolution behavior of the preparation is not affected by mechanical stress in the gastrointestinal tract. Another object of the invention is to provide a method for producing the same.

### Means for Solving the Problems

The present inventors made intensive studies in order to achieve the above objects, and as a result, they found that a preparation containing a water-soluble drug, a gel-forming polymer, and a low-melting lipophilic substance is usable as a sustained-release preparation that permits once-daily administration, the size of the preparation is small to such an extent that the preparation is easily swallowed, and the preparation can be produced by a simple method. Further, the present inventors surprisingly found that a tablet obtained by melt granulation of a water-soluble drug and a gel-forming polymer along with a low-melting substance, followed by compression of the resulting granulated product has a pH-independent matrix structure and is gradually and stably dissolved even in an environment in which the effect of mechanical stress is strong such as in the upper gastrointestinal tract after a meal, and thus, the invention has been completed.

That is, the invention is directed to:
1. a sustained-release preparation, characterized by comprising a water-soluble drug, a gel-forming polymer, and a low-melting lipophilic substance;
2. a sustained-release preparation, characterized by being produced by melt granulation of a water-soluble drug, a gel-forming polymer, and a low-melting lipophilic substance;
3. the sustained-release preparation according to item 2, wherein the melt granulation is fluidized bed melt granulation;
4. the sustained-release preparation according to any one of items 1 to 3, wherein the amount of the gel-forming polymer is from 40 to 80% by mass of the total mass of the preparation;
5. the sustained-release preparation according to any one of items 1 to 4, wherein the amount of the low-melting lipophilic substance is from 2 to 30% by mass of the total mass of the preparation;
6. the sustained-release preparation according to any one of items 1 to 5, wherein the amount of the water-soluble drug, gel-forming polymer, and low-melting lipophilic substance is from 95.0 to 99.9% by mass of the total mass of the preparation;
7. the sustained-release preparation according to any one of items 1 to 6, wherein the gel-forming polymer is one or more members selected frommethyl cellulose, hydroxypropyl cellulose, carmellose sodium, povidone, polyvinyl alcohol, and carboxy vinyl polymer;
8. the sustained-release preparation according to any one of items 1 to 7, wherein the gel-forming polymer is hydroxypropyl cellulose;
9. the sustained-release preparation according to any one of items 1 to 8, wherein the gel-forming polymer has an average particle diameter of 200 µm or less;
10. the sustained-release preparation according to any one of items 1 to 9, wherein the gel-forming polymer has a viscosity of from 1000 to 40000 mPa·s;
11. the sustained-release preparation according to any one of items 1 to 10, wherein the low-melting lipophilic substance is stearyl alcohol or stearic acid;
12. the sustained-release preparation according to any one of items 1 to 11, further comprising a lubricant;
13. the sustained-release preparation according to any one of items 1 to 12, wherein the water-soluble drug has a solubility in a phosphate buffer (pH 6.8) of 300 mg/mL or more;
14. the sustained-release preparation according to any one of items 1 to 13, wherein the water-soluble drug is metformin or cevimeline hydrochloride or a hydrate thereof;
15. the sustained-release preparation according to any one of items 1 to 14, wherein the dosage form of the preparation is a tablet;
16. the sustained-release preparation according to item 15, wherein the dosage form of the preparation is a press-coated tablet;
17. the sustained-release preparation according to item 16, wherein the dosage form of the preparation is a press-coated tablet, and the blending ratio of the water-soluble drug to the gel-forming polymer in the core tablet is from 1:2 to 4:1, and the blending ratio of the water-soluble drug to the gel-forming polymer in the outer shell layer is from 1:2 to 1:6;
18. the sustained-release preparation according to any one of items 15 to 17, wherein the tablet has a diameter of 10.0 mm or less;
19. the sustained-release preparation according to any one of items 15 to 18, wherein the tablet has a hardness of from 7.0 to 15.0 kp;
20. the sustained-release preparation according to any one of items 1 to 19, wherein the preparation is for oral administration;
21. the sustained-release preparation according to any one of items 1 to 20, wherein, in the dissolution test according to Japanese Pharmacopoeia Dissolution Test Method 2, the dissolution rates of the water-soluble drug from the preparation after the lapse of 2 hours, 4 hours, and 6 hours from initiation of the dissolution test are from 5 to 60%, from 10 to 70%, and from 20 to 90%, respectively;
22. the sustained-release preparation according to any one of items 1 to 21, wherein, in the dissolution test according to Japanese Pharmacopoeia Dissolution Test Method 2, the dissolution rates of the water-soluble drug from the preparation after the lapse of 3 hours and 6 hours from initiation of the dissolution test are from 15 to 45% and from 30 to 65%, respectively; and
23. a method for producing asustained-release preparation,
characterized by comprising the step of subjecting a water-soluble drug, a gel-forming polymer, and a low-melting lipophilic substance to melt granulation.

### Advantage of the Invention

The sustained-release preparation of the invention stably releases a drug over time when it is orally administered, and therefore, it can be used as a sustained-release preparation which can maintain an effective blood drug concentration by once-daily administration. In particular, when the sustained-release preparation of the invention is orally administered, the preparation does not easily disintegrate even if the preparation receives mechanical stress (hereinafter also referred to as mechanical shear) by gastrointestinal motility in the upper gastrointestinal tract or does not promptly dissolve in the body. Therefore, the blood drug concentration is not increased by release of a large amount of the drug in the body in a short time, and thus, the preparation can also contribute to a reduction in side effects. In particular, when the dosage form of the sustained-release preparation of the invention is a press-coated tablet, the preparation exhibits substantially zero-order release dissolution behavior whereby the drug is released at a substantially constant rate.
Further, when the dosage form of the sustained-release preparation of the invention is a tablet, the preparation has a favorable adaptability to production such that the fluidity thereof at the time of compression (tableting) is improved, etc. , and has a sufficient hardness. In particular, the sustained-release preparation of the invention has an advantage in that the hardness thereof does not decrease even if a lubricant such as magnesium stearate is blended therein.
Further, the use of melt granulation, particularly fluidized bed melt granulation in the production of the sustained-release preparation of the invention increases the yield of granulated product, reduces the proportion of aggregates or fine powder in the particle size distribution, and can greatly reduce the granulation time.
Further, the invention contributes to reduction in the size of a preparation, particularly reduction in the size of a tablet, and can make the formulation components simple. Consequently, the invention has the advantage of facilitating the selection of additives when a variety of drugs are used, because incompatibility between additives and drugs is less.

### Brief Description of the Drawings

[Fig. 1] Fig. 1 shows the drug dissolution behavior from a preparation when the low-melting lipophilic substance was changed.
[Fig. 2] Fig. 2 shows the effect of mechanical shear on drug dissolution from the tablet of Example 5.
[Fig. 3] Fig. 3 shows the effect of mechanical shear on drug dissolution from the tablet of Comparative Example 2.
[Fig. 4] Fig. 4 shows the effect of mechanical shear on drug dissolution from the tablet of Comparative Example 3.
[Fig. 5] Fig. 5 shows the effect of mechanical shear on drug dissolution behavior and drug dissolution from the tablet of Example 6.
[Fig. 6] Fig. 6 shows the effect of mechanical shear on drug dissolution from the tablet of Comparative Example 4.
[Fig. 7] Fig. 7 shows the change in plasma drug concentration (mean ± S.E., n=5) after the tablet of Example 5 was orally administered to a dog under fasted or fed conditions.
[Fig. 8] Fig. 8 shows the drug dissolution behavior from press-coated tablets.
[Fig. 9] Fig. 9 shows the drug dissolution behavior from the press-coated tablet of Example 10.

### Best Mode for Carrying Out the Invention

The invention relates to a preparation obtained by granulating a water-soluble drug as an active ingredient, and a gel-forming polymer and a low-melting lipophilic substance as additives by melt granulation, and tableting the resulting granulated product as needed.
The water-soluble drug according to the invention refers to a drug which is very soluble in water or a drug which is freely soluble in water. The terms "very soluble in water" and "freely soluble in water" as used herein have the same meanings as the terms "very soluble" and "freely soluble" described in the Japanese Pharmacopoeia Fifteenth Edition. In the invention, even if the water-soluble drug according to the invention is a drug having a solubility in a phosphate buffer (pH 6.8) described in the Japanese Pharmacopoeia Fifteenth Edition of 300 mg/mL or more, the drug can be stably released in the body. Further, even a drug having a solubility of 400 mg/mL or more can be applied to the sustained-release preparation of the invention. Examples of drugs having a solubility in a phosphate buffer (pH 6.8) of 300 mg/mL or more include roxatidine acetate hydrochloride, potassium citrate, metoprolol, choline theophylline, levocarnitine chloride, sodium valproate, chlorpromazine hydrochloride, diltiazem hydrochloride, oxybutynin hydrochloride, metformin, and cevimeline hydrochloride or a hydrate thereof. As the water-soluble drug according to the invention, metformin or cevimeline hydrochloride or a hydrate thereof is particularly preferred.

The amount of the water-soluble drug in the sustained-release preparation of the invention is preferably from 5 to 50% by mass, more preferably from 10 to 35% by mass of the total mass of the preparation.
The gel-forming polymer according to the invention refers to a polymeric substance which swells and turns into a gel when it comes into contact with water. Examples of such a substance include cellulose derivatives such as methyl cellulose, hydroxypropyl cellulose (HPC), hypromellose (hydroxypropylmethyl cellulose (HPMC)), and carmellose sodium (carboxymethyl cellulose sodium); macromolecular polysaccharides such as carrageenan, guar gum, and gum arabic; povidone (polyvinylpyrrolidone), polyvinyl alcohol, polyethylene oxides, and carboxy vinyl polymers. These may be used alone or in combination of two or more kinds. As the gel-forming polymer according to the invention, one or more members selected frommethyl cellulose, hydroxypropyl cellulose, carmellose sodium, povidone, polyvinyl alcohol, and a carboxy vinyl polymer are preferred, and hydroxypropyl cellulose is particularly preferred.
Further, when the gel-forming polymer according to the invention has an average particle diameter of 200 µm or less, the sustained-release effect is increased. In the invention, when the average particle diameter of the gel-forming polymer is 150 µm or less, the sustained-release effect is further increased, which is therefore preferred.
Further, the gel-forming polymer according to the invention has a viscosity of generally from 500 to 100000 mPa·s, preferably from 1000 to 40000 mPa·s when the measurement is performed at 1% gel-forming polymer in water at 20°C. When the viscosity of the gel-forming polymer falls within the range of from 1000 to 40000 mPa·s, the dissolution of the water-soluble drug from the sustained-release preparation of the invention can be effectively controlled, which is therefore preferred.
Further, when the gel-forming polymer according to the invention is hydroxypropyl cellulose, it is also possible to control the release profile of the drug by appropriately selecting several types of hydroxypropyl cellulose having different viscosities and suitably combining them.
The amount of the gel-forming polymer in the sustained-release preparation of the invention is preferably from 40 to 80% by mass, more preferably from 50 to 80% by mass of the total mass of the preparation.
The blending ratio of the water-soluble drug to the gel-forming polymer according to the invention is preferably from 1:1 to 1:5, more preferably from 1:1.5 to 1:4.
The low-melting lipophilic substance according to the invention refers to a substance which has a melting point of from 30 to 100°C and has the property (lipophilicity) that the amount of water required for dissolving 1 g of the substance is 100 mL or more. In the invention, the low-melting lipophilic substance preferably has a melting point of from 50 to 90°C. Examples of such a low-melting lipophilic substance include stearyl alcohol, stearic acid; medium-chain fatty acid esters such as glyceryl monostearate, and glyceryl tristearate; and hydrogenated oils. As the low-melting lipophilic substance according to the invention, stearyl alcohol or stearic acid is preferred, and stearyl alcohol is more preferred. In the invention, by selecting stearyl alcohol or stearic acid as the low-melting lipophilic substance, the sustained-release preparation of the invention does not easily disintegrate even by gastro intestinal motility in the upper gastro intestinal tract after it is orally administered, and can stably release the drug from the surface of the preparation over time. In the invention, as the low-melting lipophilic substance, stearyl alcohol and stearic acid may be used in combination.
The amount of the low-melting lipophilic substance in the sustained-release preparation of the invention is preferably from 8 to 30% by mass, more preferably from 10 to 20% by mass of the total mass of the preparation.
When the dosage form of the sustained-release preparation of the invention is a tablet, a lubricant can be blended in addition to the water-soluble drug, gel-forming polymer, and low-melting lipophilic substance, however, by selecting stearic acid or stearyl alcohol as the low-melting lipophilic substance according to the invention, a decrease in the hardness of the tablet due to the lubricant can be prevented. The lubricant as used herein refers to a substance to be added for preventing failure in tableting such as sticking which occurs at the time of compression. As the lubricant according to the invention, magnesium stearate, sodiumstearylfumarate, talc, and synthetic aluminum silicate can be exemplified, and magnesium stearate is preferred.
In the sustained-release preparation of the invention, in addition to the water-soluble drug, gel-forming polymer, and low-melting lipophilic substance, a pharmaceutical additive commonly used in the production of a pharmaceutical preparation such as a coloring agent can be blended as needed within the range that does not deteriorate the effects of the invention.
Examples of the coloring agent in the invention include food coloring agents.
Further, as another pharmaceutical additive, a binder, a surfactant, a pH adjusting agent or the like can be appropriately added as needed.
Further, in the sustained-release preparation of the invention, a diluent such as lactose, cornstarch, crystalline cellulose, potato starch, synthetic aluminum silicate, magnesium aluminometasilicate, calcium phosphate, or calcium carbonate may be blended within the range that does not deteriorate the effects of the invention. However, the preparation of the invention can be produced without blending an excipient, a disintegrant, or the like. Reduction in addition of components other than the water-soluble drug, gel-forming polymer, and low-melting lipophilic substance, particularly an excipient and a disintegrant to the sustained-release preparation of the invention as much as possible can reduce the size of the preparation, the number of production steps, and the production cost, and is therefore preferred.
In the sustained-release preparation of the invention, the total mass of the water-soluble drug, gel-forming polymer, and low-melting lipophilic substance is generally from 95.0 to 99. 9% by mass, preferably from 97.0 to 99.0% by mass of the total mass of the preparation. Further, when the dosage form of the preparation of the invention is a tablet, the total mass of the water-soluble drug, gel-forming polymer, low-melting lipophilic substance, and lubricant contained in the sustained-release preparation of the invention may be from 98.0 to 100.0% by mass of the total mass of the preparation, and a sustained-release preparation composed of only the water-soluble drug, gel-forming polymer, low-melting lipophilic substance, and lubricant can also be prepared.
The dosage form of the sustained-release preparation of the invention is a solid preparation. The administration route of the sustained-release preparation of the invention may be oral administration or parenteral administration, however, oral administration is preferred. As the dosage form of the sustained-release preparation of the invention, a tablet, a granule, or a powder is preferred, and a tablet is more preferred. Further, it is also possible to form an encapsulated formulation by filling the granule or powder in a capsule.
Hereinafter, a method for producing the sustained-release preparation of the invention is described.
The method for producing the sustained-release preparation of the invention is characterized by comprising the step of subj ecting the water-soluble drug, gel-forming polymer, and low-melting lipophilic substance to melt granulation. Selection of melt granulation as the granulation method is preferred because, as compared with the case of using other granulation methods, the yield is higher, the variation in particle size is smaller, and the granulation time is shorter and therefore the efficiency is higher. Examples of the melt granulation method include melt granulation with stirring and fluidized bed melt granulation, however, in the invention, granulation through fluidized bed melt granulation is preferred from the viewpoint of operability and efficiency at the time of production, and improvement of tablet strength due to the relatively small specific volume of the resulting granulated powder. Further, in the granulated product, a lubricant or another additive is appropriately added as needed, and the resulting mixture is compression-molded at a suitable tableting pressure using a common method, whereby a tablet can be produced.
When the dosage form of the sustained-release preparation of the invention is a tablet, the preparation can also be in the form of a press-coated tablet, a multilayered tablet, or a shaped tablet.
The press-coated tablet refers to a tablet having a core tablet in the center thereof, the periphery of which is coated with an outer shell layer. The press-coated tablet can be produced by, for example, forming the core tablet by tableting in advance and tableting the core tablet along with outer shell layer components. When the dosage form of the sustained-release preparation of the invention is a press-coated tablet, by blending, in the core tablet and the outer shell layer, different types of granulated product each containing different components or each containing the same components at different blending ratios, a preparation which exhibits favorable dissolution behavior can be prepared.
The multilayered tablet is a tablet obtained by laminating two or three layers of powder particle materials each containing different components or containing the same components at different blending ratios, followed by tableting.
Further, the sustained-release preparation according to the invention may be in the form of a shaped tablet in an elliptical, rhombic, or triangular shape other than a common round shape.
In the case where the dosage form of the sustained-release preparation of the invention is a press-coated tablet, the blending ratios of the water-soluble drug to the gel-forming polymer in the core tablet and the outer shell layer are as follows. It is preferred that when the blending ratio of the water-soluble drug to the gel-forming polymer in the core tablet is from 1:2 to 4:1, the blending ratio of the water-soluble drug to the gel-forming polymer in the outer shell layer is from 1:2 to 1: 6. It is more preferred when the blending ratio of the water-soluble drug to the gel-forming polymer in the core tablet is from 1:1 to 3:1, the blending ratio of the water-soluble drug to the gel-forming polymer in the outer shell layer is from 1:3 to 1: 5. By blending the components in the core tablet and the outer shell layer as described above, the dissolution behavior of the drug from the preparation becomes a substantially constant rate (substantially zero-order release dissolution behavior), and a more stable drug release from the preparation can be achieved.
The sustained-release preparation of the invention can be subjected to a coating treatment by a common method. As a coating agent to be used for the coating, for example, a cellulose-based water-soluble coating can be exemplified. When the dosage form of the preparation of the invention is a press-coated tablet, the core tablet and/or outer shell layer of the press-coated tablet may be subjected to film-coating of at least one layer. The sustained-release preparation of the invention can stably release the drug over time with respect to dissolution of the water-soluble drug from the preparation. Specifically, in the sustained-release preparation of the invention, it is preferred that the dissolution rates of the water-soluble drug from the preparation in the dissolution test according to Dissolution Test Method 2 described in the Japanese Pharmacopoeia Fifteenth Edition after the lapse of 2 hours, 4 hours, and 6 hours from the initiation of the dissolution test are from 5 to 60%, from 10 to 70%, and from 20 to 90%, respectively, or after the lapse of 3 hours and 6 hours from the initiation of the dissolution test are from 15 to 45% and from 30 to 65%, respectively. It is more preferred that the dissolution rates after the lapse of 2 hours, 3 hours, 4 hours, and 6 hours from the initiation of the dissolution test are from 5 to 60%, from 15 to 45%, from 10 to 70%, and from 30 to 65%, respectively. It is particularly preferred that the dissolution rates after the lapse of 2 hours, 3 hours, 4 hours, 6 hours, and 12 hours from the initiation of the dissolution test are from 5 to 40%, from 15 to 45%, from 20 to 60%, from 30 to 65%, and from 60 to 95%, respectively.

Further, the sustained-release preparation of the invention can be reduced in size to such an extent that the preparation is easily taken. When the dosage form of the sustained-release preparation of the invention is a tablet, the tablet has a diameter of preferably 10.0 mm or less, more preferably 9.0 mm or less.
When the dosage form of the sustained-release preparation of the invention is a tablet, the tablet has a hardness of preferably 7.0 kp or more, more preferably from 7.0 to 15.0 kp in the case where the tablet has a diameter of 10.0 mm or less.

### Examples

Hereinafter, the invention is described in more detail with reference to Examples. However, the invention is by no means limited to these Examples.

### (Example 1)

93.5 g of cevimeline hydrochloride hydrate, 230 g of hydroxypropyl cellulose (H fine powder) (HPC-H fine powder, Nippon Soda Co., Ltd.), and 40 g of stearic acid (stearic acid NAA-180P, NOF Corporation) were mixed, and the resulting mixture was subjected to melt granulation using a fluidized bed granulator (Multiplex, MP-01, Powrex Corporation) at an inlet air temperature of 80°C and an airflow rate of 50 to 60 m²/h. At this time, granulation was completed at the time when the product temperature of the preparation became around the melting point of stearic acid (about 72°C).

### (Comparative Example 1)

93.5 g of cevimeline hydrochloride hydrate, 270 g of hydroxypropyl cellulose (H fine powder) were mixed, and the resulting mixture was subjected to wet granulation using a fluidized bed granulator (Flow-Coater Mini, FLO-mini, Freund Industrial Co., Ltd.) at an inlet air temperature of 90°C and using water as a binder solution at a binder solution spray rate of 1 g/min. After completion of granulation, the resulting granulated product was dried in the fluidized bed granulator until the temperature became around 35°C. Thereafter, the granulated product was further dried in an oven at 40°C for 12 hours to sufficiently remove water.
The yield and particle size distribution of the granulated product obtained by granulation using each granulation method and the granulation time therefor are shown in Table 1. As compared with the case of using a wet granulation method, in the case of using a melt granulation method, the yield was higher, the proportion of aggregates (850 µm or more (retained on a 18 mesh sieve)) and fine powder (less than 106 µm (passing through a 140 mesh sieve)) in the particle size distribution was less, and the granulation time could be greatly reduced. These results showed that as compared with the case where granulation was performed by wet granulation, in the case where granulation was performed by melt granulation, the yield was higher, the variation in particle size was smaller, and the granulation time was shorter and therefore the efficiency was higher.

**Table 1**

| | Results | | | | |
|---|---|---|---|---|---|
| | Yield (%) | Particle size distribution (%) | | | Granulation time (min) |
| | | ≥ 850 µm | 106 µm- 850 µm | < 106 µm | |
| Example 1 | 90.2 | 0 | 90.9 | 9.1 | 30 |
| Comparative Example 1 | 76.6 | 12.8 | 70.0 | 17.2 | 120 |

### (Example 2)

All the components except magnesium stearate shown in the formulation of Example 2 in Table 2 were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearyl alcohol (about 60°C). As the stearyl alcohol, a product from Kao Corporation was used.
Further, in this melt-granulated powder, magnesium stearate (magnesium stearate, Nitto Kasei Co., Ltd.) was mixed, and the resulting mixture was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Example 3)

All the components except magnesium stearate shown in the formulation of Example 3 in Table 2 were mixed, and the resulting mixture was processed in the same manner as in Example 2, whereby a melt-granulated powder was obtained. However, melt granulation was completed at the time when the product temperature of the preparation became around the melting point of glyceryl monostearate (around about 70°C). As the glyceryl monostearate, a product from Riken Vitamin Co., Ltd. was used. Further, in this melt-granulated powder, magnesium stearate (magnesium stearate, Nitto Kasei Co., Ltd.) was mixed, and the resulting mixture was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Example 4)

All the components except magnesium stearate shown in the formulation of Example 4 in Table 2 were mixed, and the resulting mixture was processed in the same manner as in Example 2, whereby a melt-granulated powder was obtained. However, melt granulation was completed at the time when the product temperature of the preparation became around the melting point of stearic acid (about 72°C). Further, in this melt-granulated powder, magnesium stearate (magnesium stearate, Nitto Kasei Co., Ltd.) was mixed, and the resulting mixture was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

**Table 2**

| Component | Formulation (mg) | | |
|---|---|---|---|
| | Example 2 | Example 3 | Example 4 |
| Cevimeline hydrochloride hemihydrate | 93.5 | 93.5 | 93.5 |
| (in terms of cevimeline hydrochloride) | (90.0) | (90.0) | (90.0) |
| Hydroxypropyl cellulose | 230.0 | 230.0 | 230.0 |
| Stearyl alcohol | 40.0 | - | - |
| Glyceryl monostearate | - | 40.0 | - |
| Stearic acid | - | - | 40.0 |
| Magnesium stearate | 3.0 | 3.0 | 3.0 |
| Total | 366.5 | 366.5 | 366.5 |
| Pestle diameter (mm) | 10.0 | 10.0 | 10.0 |

### (Test Example 1)

The powder physical properties of the respective melt-granulated powders of Examples 2 to 4 were measured (Table 3). 100 g of each granulated powder was weighed and shaken for 10 minutes using sieves of different mesh sizes (mesh sizes: 1000 µm and 106 µm). The weight of each fraction was measured and the particle size distribution (%) of each granulated powder was calculated. Further, the repose angles were measured using a powder tester, and the average particle diameters were measured by laser diffractometry. The properties of the respective melt-granulated powders are shown in Table 3. In all the granulated powders, a fraction having a size of 1000 µm or more was not observed, and a fraction having a size of less than 106 µm was less than 15%, and therefore, favorable melt-granulated powders were obtained.

**Table 3**

| Item | | Example 2 | Example 3 | Example 4 |
|---|---|---|---|---|
| Particle size distribution (%) | ≥ 1000 µm | 0 | 0 | 0 |
| | 106 µm - 1000 µm | 91.6 | 93.6 | 87.6 |
| | < 106 µm | 8.4 | 6.4 | 12.4 |
| Repose angle (degree) | | 39 | 40 | 39 |
| Average particle diameter (nm) | | 183 | 169 | 118 |

### (Test Example 2)

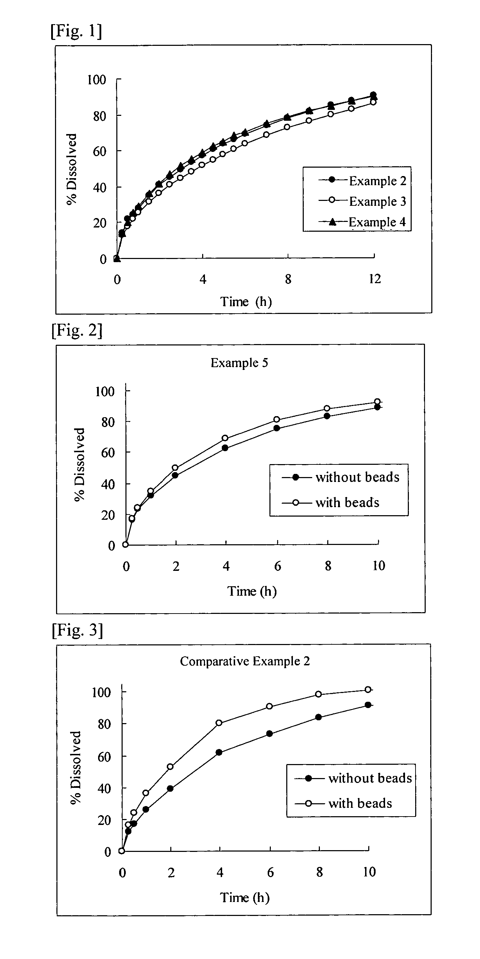

A test (dissolution test) for determining the drug dissolution rates from the tablets obtained by tableting the respective melt-granulated powders of Examples 2 to 4 was performed. The test method was in accordance with Dissolution Test Method 2 described in the Japanese Pharmacopoeia Fifteenth Edition. The test was performed by the paddle method (paddle rotation speed: 50 rpm) using 900 mL of water at 37°C as a solution for dissolution. The results are shown in Fig. 1. All the tablets of Examples 2 to 4 exhibited dissolution behavior such that the dissolution rates after the lapse of 4 hours and 12 hours from the initiation of the dissolution test were from 20 to 60%, and from 60 to 95%, respectively.

### (Example 5)

93.5 g of cevimeline hydrochloride hydrate, 230 g of hydroxypropyl cellulose, and 40 g of stearic acid were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearic acid (about 72°C). Further, in this melt-granulated powder, 3 g of magnesium stearate was mixed, and the resulting mixture was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Comparative Example 2)

A melt-granulated powder and a tablet were obtained in the same manner as in Example 5 using 40 g of polyethylene glycol 6000 (Macrogol 6000, NOF Corporation) which is widely used as a low-melting substance in place of stearic acid in Example 5. However, melt granulation was completed at the time when the product temperature of the preparation became around the melting point of polyethylene glycol 6000 (about 65°C).

### (Comparative Example 3)

A melt-granulated powder and a tablet were obtained in the same manner as in Example 5 using 180 g of ethyl cellulose (ETHOCEL STD 7 FP, Nisshin & Co. , Ltd.) which is a water-insoluble polymer, 15 g of carnauba wax (Polishing Wax 103, Freund Industrial Co., Ltd.) which is a wax base, and 18 g of sucrose fatty acid ester (Surfhope J-1803, Mitsubishi Chemical Foods Co., Ltd.) in place of the gel-forming polymer (hydroxypropyl cellulose) in Example 5. Incidentally, as the low-melting lipophilic substance, stearic acid was used in the same manner as in Example 4.

**Table 4**

| Component | Formulation (mg) | | |
|---|---|---|---|
| | Example 5 | Comparative Example 2 | Comparative Example 3 |
| Cevimeline hydrochloride hemihydrate (in terms of cevimeline hydrochloride) | 93.5 (90.0) | 93.5 (90.0) | 93.5 (90.0) |
| Hydroxypropyl cellulose | 230.0 | 230.0 | - |
| Ethyl cellulose | - | - | 180.0 |
| Carnauba wax | - | - | 15.0 |
| Stearic acid | 40.0 | - | 60.0 |
| Polyethylene glycol 6000 | - | 40.0 | - |
| Sucrose fatty acid ester | - | - | 18.0 |
| Magnesium stearate | 3.0 | 3.0 | 3.0 |
| Total | 366.5 | 366.5 | 369.5 |
| Pestle diameter (mm) | 10.0 | 10.0 | 10.0 |

### (Test Example 3)

In order to evaluate the strength of a tablet against mechanical shear which acts on a preparation, a dissolution test (beads method) was performed for the tablets of Example 5, Comparative Example 2 and Comparative Example 3. The beads method is a test method for evaluating the effect of mechanical shear on drug dissolution from a tablet by adding 800 beads (diameter: 6.4 mm, specific gravity: 1.14, made of Nylon 66) per vessel. The test was performed by the paddle method (paddle rotation speed: 50 rpm) using 800 mL of water at 37°C as a solution for dissolution.
The results are shown in Figs. 2 to 4 and Table 5. The effect of mechanical shear was smaller and the tablet has a more favorable strength in the case of the formulation using stearic acid having a high lipophilicity as a low-melting substance (Example 5) than in the case of the formulation using polyethylene glycol 6000 having a high water solubility as a low-melting substance (Comparative Example 2). Further, even when ethyl cellulose which is an insoluble polymer and carnauba wax which is a wax base were used as sustained-release bases and these components were combined with stearic acid (Comparative Example 3), a favorable tablet strength which was obtained by the combination of a gel-forming polymer with stearic acid (Example 5) was not obtained.

**Table 5**

| Time (h) | Difference in dissolution rate between with and without beads* (%) | | |
|---|---|---|---|
| | Example 5 | Comparative Example 2 | Comparative Example 3 |
| 0.5 | 1.1 | 6.7 | 3.0 |
| 1 | 3.0 | 10.1 | 4.6 |
| 2 | 4.8 | 14.2 | 8.7 |
| 4 | 6.3 | 18.3 | 19.3 |
| 6 | 5.9 | 16.7 | 23.8 |
| 8 | 5.3 | 13.9 | 18.5 |
| 10 | 3.5 | 9.7 | 9.3 |

| | | | |
|---|---|---|---|
| * : (Dissolution rate in the case of with beads) - (Dissolution rate in the case of without beads) | | | |

### (Example 6)

100 g of metformin, 210 g of hydroxypropyl cellulose, and 60 g of stearyl alcohol were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearyl alcohol (about 60°C). Further, in this melt-granulated powder, 3 g of magnesium stearate was mixed, and the resulting mixture was tableted using a single tableting machine (N-30E, Okada Seiko Co., Ltd.) at a tableting pressure of 800 kgf/pestle, whereby a tablet having a diameter of 10 mm and a mass of 373 mg was obtained.

### (Comparative Example 4)

A melt-granulated powder and a tablet were obtained in the same manner as in Example 6 using 60 g of polyethylene glycol 6000 (Macrogol 6000, NOF Corporation) which is widely used as a low-melting substance in place of stearyl alcohol in Example 6. However, melt granulation was completed at the time when the product temperature of the preparation became around the melting point of polyethylene glycol 6000 (about 65°C).

**Table 6**

| Component | Formulation (mg) | |
|---|---|---|
| | Example 6 | Comparative Example 4 |
| Metformin | 100.0 | 100.0 |
| Hydroxypropyl cellulose | 210.0 | 210.0 |
| Stearic acid | 60.0 | - |
| Polyethylene glycol 6000 | - | 60.0 |
| Magnesium stearate | 3.0 | 3.0 |
| Total | 373.0 | 373.0 |
| Pestle diameter (mm) | 10.0 | 10.0 |

### (Test Example 4)

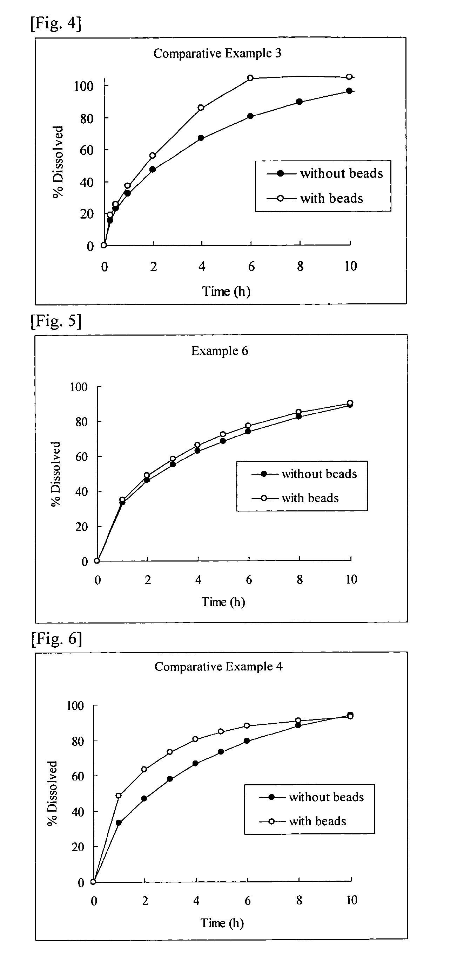

In order to evaluate the strength of a tablet against mechanical shear which acts on a preparation, a dissolution test (beads method) was performed for the tablets of Example 6 and Comparative Example 4. The beads method is a test method for evaluating the effect of mechanical shear on drug dissolution from a tablet by adding 800 beads (diameter: 6.4 mm, specific gravity: 1.14, made of Nylon 66) per vessel. The test was performed by the paddle method (paddle rotation speed: 50 rpm) using 800 mL of water at 37°C as a solution for dissolution.
The results are shown in Figs. 5 and 6 and Table 7. From Fig. 5, it was confirmed that even when metformin was used as a water-soluble drug, the resulting tablet was a stable sustained-release preparation. Further, from Fig. 5 and Fig. 6, the effect of mechanical shear was smaller and the tablet has a more favorable tablet strength in the case of the formulation using stearic acid having a high lipophilicity as a low-melting substance (Example 6) than in the case of the formulation using polyethylene glycol 6000 having a high water solubility as a low-melting substance (Comparative Example 4).

**Table 7**

| Time (h) | Difference in dissolution rate between with and without beads* (%) | |
|---|---|---|
| | Example 6 | Comparative Example 4 |
| 1 | 1.8 | 15.6 |
| 2 | 2.7 | 16.1 |
| 3 | 3.4 | 15.1 |
| 4 | 3.5 | 13.8 |
| 6 | 3.1 | 8.5 |
| 8 | 2.3 | 2.6 |

| | | |
|---|---|---|
| *: (Dissolution rate in the case of with beads) - (Dissolution rate in the case of without beads) | | |

### (Comparative Example 5)

Without using magnesium stearate, the melt-granulated powder obtained in Example 2 was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Comparative Example 6)

Without using magnesium stearate, the melt-granulated powder obtained in Example 4 was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Comparative Example 7)

Without using magnesium stearate, the melt-granulated powder obtained in Comparative Example 2 was tableted using a single tableting machine (N-30E, Okada Seiko Co., Ltd.) at a tableting pressure of 800 kgf/pestle, whereby a tablet having a diameter of 10 mm and a mass of 366.5 mg was obtained.

### (Test Example 5)

The hardness of each of the tablets of Example 2, Example 4, Comparative Example 2, Comparative Examples 5 to 7 was measured using a tablet tester (model 6D manufactured by SCHLEUNIGER). The results are shown in Table 8.

**Table 8**

| | Low-melting substance | With or without use of magnesium stearate at tableting | With or without occurrence of sticking | Hardness of tablet (kp) | Difference in hardness (kp) |
|---|---|---|---|---|---|
| Example 2 | Stearyl alcohol | With | Without | 9.7 | 2.2 |
| Comparative Example 5 | | Without | With | 11.9 | |
| Example 4 | Stearic acid | With | Without | 7.3 | 2.6 |
| Comparative Example 6 | | Without | With | 9.9 | |
| Comparative Example 2 | Polyethylene glycol 6000 | With | Without | 5.6 | 7.7 |
| Comparative Example 7 | | Without | With | 13.3 | |

In the case where a low-melting lipophilic substance such as stearyl alcohol or stearic acid was used as a low-melting substance, even if magnesium stearate was added when the preparation (tablet) was produced, no significant change in the hardness of the tablet was observed (comparison between Example 2 and Comparative Example 5, and comparison between Example 4 and Comparative Example 6). On the other hand, in the case where water-soluble polyethylene glycol 6000 was used as a low-melting substance, by adding magnesium stearate when the preparation was produced, the hardness of the tablet was significantly decreased (comparison between Comparative Example 2 and Comparative Example 7). It is necessary for the prevention of sticking at the time of tableting to add a lubricant such as magnesium stearate when a tablet is produced. Therefore, it was found that the blending of a low-melting lipophilic substance is extremely superior to the blending of a low-melting water-soluble substance also from the viewpoint of increasing the hardness of the preparation, particularly a tablet.

### (Test Example 6)

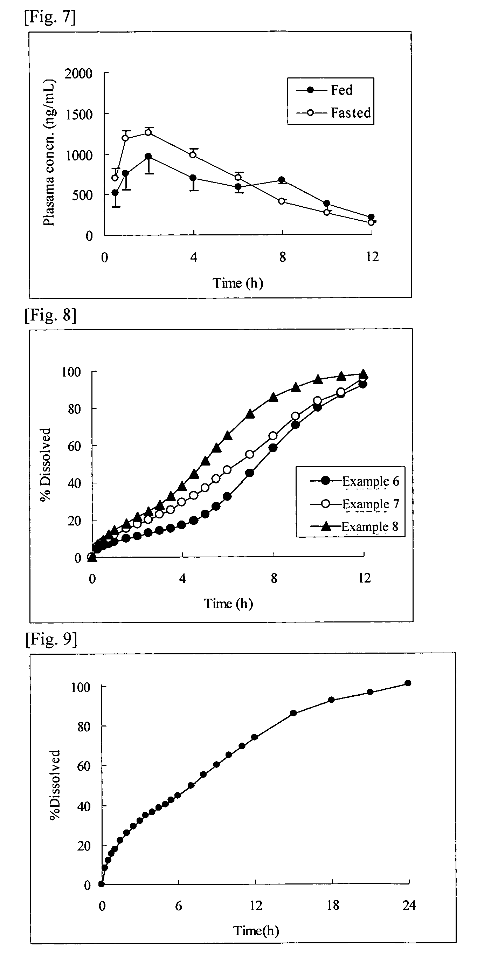

The tablet of Example 5 was orally administered to a dog (beagle) under fasted or fed conditions, and the effect of feeding on disintegration of the tablet was evaluated. The results of the change in plasma drug concentration are shown in Fig. 7 and the pharmacokinetic parameters are shown in Table 9. As a result, the maximum blood concentration (Cmax) and the area under the curve of concentration (AUC_{0-∞}) were almost equivalent between the fasted conditions and the fed conditions, and it was found that the tablet does not disintegrate even in an environment in which the mechanical stress is strong such as in the upper gastrointestinal tract after feeding, that is, the preparation of the invention has a strength such that it can sufficiently withstand mechanical shear in the actual gastrointestinal tract.

**Table 9 Pharmacokinetic parameters (mean ± S.E., n=5)**

| Formulations | AUC_{0-∞} (ng/mL) | Cₘₐₓ (ng/mL) | Tₘₐₓ (hr) | MRT (h) |
|---|---|---|---|---|
| Administration under fed conditions | 8112 ± 572 | 1260 ± 82 | 3.0 ± 1.3 | 5.8 ± 1.0 |
| Administration under fasted conditions | 8334 ± 735 | 1210±102 | 1.8 ± 0.3 | 4.6 ± 0.2 |

### (Example 7)

All the components of the core tablet shown in the formulation of Example 7 in Table 10 except magnesium stearate were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearic acid (around about 72°C).
Further, in this melt-granulated powder, magnesium stearate was mixed, and the resulting mixture was tableted using a single tableting machine (N-30E, Okada Seiko Co., Ltd.) at a tableting pressure of 800 kgf/pestle, whereby a tablet (core tablet) having a diameter of 6.7 mm was obtained.
Further, all the components of the outer shell layer shown in the formulation of Example 7 in Table 10 except magnesium stearate were mixed, and the resulting mixture was subjected to melt granulation in the same manner as the core tablet components, whereby a melt-granulated powder was obtained. In the resulting melt-granulated powder, magnesium stearate was mixed, and the resulting mixture was tableted together with the core tablet using a single tableting machine (N-30E, Okada Seiko Co., Ltd.) at a tableting pressure of 800 kgf/pestle, whereby a press-coated tablet having a diameter of 9. 0 mm was obtained.

### (Example 8)

All the components of the core tablet shown in the formulation of Example 8 in Table 10 except magnesium stearate were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearic acid (about 72°C).
Further, in this melt-granulated powder, magnesium stearate was mixed, and the resulting mixture was tableted using a single tableting machine (N-30E, Okada Seiko Co., Ltd.) at a tableting pressure of 800 kgf/pestle, whereby a tablet (core tablet) having a diameter of 6.7 mm was obtained.
Further, all the components of the outer shell layer shown in the formulation of Example 8 in Table 10 except magnesium stearate were mixed, and the resulting mixture was subjected to melt granulation in the same manner as the core tablet components, whereby a melt-granulated powder was obtained. In the resulting melt-granulated powder, magnesium stearate was mixed, and the resulting mixture was tableted together with the core tablet using a single tableting machine (N-30E, Okada Seiko Co., Ltd.) at a tableting pressure of 800 kgf/pestle, whereby a press-coated tablet having a diameter of 9.0 mm was obtained.

### (Example 9)

All the components of the core tablet shown in the formulation of Example 9 in Table 10 except magnesium stearate were mixed, and the resulting mixture was subjected to melt granulation using a fluidized bed granulator (Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearic acid (about 72°C).
Further, in this melt-granulated powder, magnesium stearate was mixed, and the resulting mixture was tableted using a single tableting machine (N-30E, Okada Seiko Co., Ltd.) at a tableting pressure of 800 kgf/pestle, whereby a tablet (core tablet) having a diameter of 6.0 mm was obtained.
Further, the components of the outer shell layer shown in the formulation of Example 9 in Table 10 were mixed, and the resulting mixture was subjected to melt granulation in the same manner as the core tablet components, whereby a melt-granulated powder was obtained. In the resulting melt-granulated powder, magnesium stearate was mixed, and the resulting mixture was tableted together with the core tablet using a single tableting machine (N-30E, Okada Seiko Co., Ltd.) at a tableting pressure of 800 kgf/pestle, whereby a press-coated tablet having a diameter of 8.5 mm was obtained.

**Table 10 Study for formulation of press-coated tablet**

| Components | | Formulation (mg) | | |
|---|---|---|---|---|
| | | Example 7 | Example 8 | Example 9 |
| Cevimeline hydrochloride hemihydrate (in terms of cevimeline hydrochloride) | Core tablet | 72.7 | 62.3 | 62.3 |
| | | (70.0) | (60.0) | (60.0) |
| | Outer shell layer | 20.8 | 31.2 | 31.2 |
| | | (20.0) | (30.0) | (30.0) |
| | Press-coated | 93.5 | 93.5 | 93.5 |
| | tablet | (90.0) | (90.0) | (90.0) |
| Hydroxypropyl cellulose | Core tablet | 10.0 | 20.0 | 5.0 |
| | Outer shell layer | 160.0 | 150.0 | 120.0 |
| | Press-coated tablet | 170.0 | 170.0 | 125.0 |
| Stearic acid | Core tablet | 10.0 | 10.0 | 5.0 |
| | Outer shell layer | 20.0 | 20.0 | 17.5 |
| | Press-coated tablet | 30.0 | 30.0 | 22.5 |
| Magnesium stearate | Core tablet Outer shell layer | 1.0 | 1.0 | 1.0 |
| | | 2.0 | 2.0 | 2.0 |
| | Press-coated tablet | 3.0 | 3.0 | 3.0 |
| Total | Core tablet Outer shell layer | 93.7 | 93.3 | 73.3 |
| | | 202.8 | 203.2 | 170.7 |
| | Press-coated tablet | 296.5 | 296.5 | 244.0 |
| Pestle diameter (mm) | Core tablet | 6.7 | 6.7 | 6.0 |
| | Press-coated tablet | 9.0 | 9.0 | 8.5 |

### (Test Example 7)

The powder physical properties of the respective melt-granulated powders for core tablets and for outer shell layers of Example 7 to 9 were measured. The results are shown in Table 11. 100 g of each granulated powder was weighed and shaken for 10 minutes using sieves of different mesh sizes (mesh sizes: 1 mm, 500 µm, 355 µm, 250 µm, 150 µm, 106 µm, and 75 µm). The weight of each fraction was measured and the particle size distribution (%) of each granulated powder was calculated. Further, the repose angles were measured using a powder tester. In all the granulated powders, a fraction having a size of 1000 µm or more was not observed, and a fraction having a size of 106 µm or less was from about 5 to 20%, and therefore, favorable granulated powders were obtained.

**Table 11**

| Item | | Example 7 | | Example 8 | | Example 9 | |
|---|---|---|---|---|---|---|---|
| | | Core | Outer shell layer | Core | Outer shell layer | Core | Outer shell layer |
| Particle size distribution (%) | ≥ 1000 µm | 0 | 0 | 0 | 0 | 0 | 0 |
| | 106 µm -1000 µm | 96.4 | 81.4 | 93.2 | 83.1 | 91.3 | 77.7 |
| | ≤ 106 µm | 3.6 | 18.6 | 6.8 | 16.9 | 8.7 | 22.3 |
| Repose angle (degree) | | 38 | 41 | 39 | 39 | 39 | 41 |

### (Test Example 8)

A test (dissolution test) for determining the drug dissolution rates from the press-coated tablets of Examples 7 to 9 was performed. The test method was in accordance with Dissolution Test Method 2 described in the Japanese Pharmacopoeia Fifteenth Edition. The test was performed by the paddle method (paddle rotation speed: 50 rpm) using 900 mL of water at 37°C as a solution for dissolution. The results are shown in Fig. 8. It was found that all the preparations of Examples stably released the drug over time. In particular, Example 8 released the drug at a substantially constant rate (substantially zero-order release dissolution behavior), and therefore exhibited extremely ideal drug release (dissolution) behavior. It was also found that by suitably combining a gel-forming polymer with a low-melting substance and forming a press-coated tablet, the diameter of the tablet could be reduced to 8.5 mm to 9 mm.

### (Example 10)

All the components of the core tablet shown in the formulation of Example 10 in Table 12 except magnesium aluminometasilicate and magnesium stearate were mixed, and the resulting mixture was subjected to melt granulation using a fluidized bed granulator(Multiplex, MP-01, Powrex Corporation) at an inlet air temperature of 80°C and an airflow rate of 50 to 60 m²/h, whereby a melt-granulated powder was obtained. At this time, granulation was completed at the time when the product temperature of the preparation became around the melting point of stearyl alcohol (around about 60°C).
Further, in this melt-granulated powder, magnesium aluminometasilicate and magnesium stearate were mixed, and the resulting mixture was tableted using a single tableting machine (N-30E, Okada Seiko Co., Ltd.) at a tableting pressure of 800 kgf/pestle, whereby a tablet (core tablet) having a diameter of 6.0 mm was obtained.
Further, all the components of the outer shell layer shown in the formulation of Example 10 in Table 12 except magnesium aluminometasilicate and magnesium stearate were mixed, and the resulting mixture was subjected to melt granulation in the same manner as the core tablet components, whereby a melt-granulated powder was obtained. In the resulting melt-granulated powder, magnesium aluminometasilicate and magnesium stearate were mixed, and the resulting mixture was tableted together with the core tablet using a single tableting machine (N-30E, Okada Seiko Co. , Ltd.) at a tableting pressure of 800 kgf/pestle, whereby a press-coated tablet having a diameter of 10.0 mm was obtained.

**Table 12**

| Components of Example 10 | | Formulation (mg) |
|---|---|---|
| Cevimeline hydrochloride hemihydrate (in terms of cevimeline hydrochloride) | Core tablet | 41.6 (40.0) |
| | Outer shell layer | 51.9 (50.0) |
| | Press-coated tablet | 93.5 (90.0) |
| Hydroxypropyl cellulose | Core tablet | 20.8 |
| | Outer shell layer | 202.0 |
| | Press-coated tablet | 222.8 |
| Stearyl alcohol | Core tablet | 8.5 |
| | Outer shell layer | 34.6 |
| | Press-coated tablet | 43.1 |
| Magnesium aluminometasilicate | Core tablet | 0.7 |
| | Outer shell layer | 3.0 |
| | Press-coated tablet | 3.7 |
| t Magnesium stearate | Core table | 0.4 |
| | Outer shell layer | 1.5 |
| | Press-coated tablet | 1.9 |
| Total | Core tablet | 72.0 |
| | Outer shell layer | 293.0 |
| | Press-coated tablet | 365.0 |
| Pestle diameter (mm) | Core tablet | 6.0 |
| | Outer shell layer | 10.0 |

### (Test Example 9)

A test (dissolution test) for determining the drug dissolution rate from the press-coated tablet of Example 10 was performed. The test method was in accordance with Dissolution Test Method 2 described in the Japanese Pharmacopoeia Fifteenth Edition. The test was performed by the paddle method (paddle rotation speed: 50 rpm) using 900 mL of water at 37°C as a solution for dissolution. The results are shown in Fig. 9. The preparation of Example 10 released the drug at a substantially constant rate (substantially zero-order release dissolution behavior), and therefore exhibited extremely ideal drug release (dissolution) behavior.

### Industrial Applicability

The sustained-release preparation of the invention exhibited stable drug dissolution behavior without being affected by variation in the dissolution conditions. Further, the sustained-release preparation of the invention is small such that the size does not cause difficulty in taking the preparation and therefore improves medication compliance. Accordingly, the preparation of the invention is useful particularly as a sustained-release preparation containing a water-soluble drug.

## Claims

1. A sustained-release preparation, **characterized by** comprising a water-soluble drug, a gel-forming polymer, and a low-melting lipophilic substance.

2. A sustained-release preparation, **characterized by** being produced by melt granulation of a water-soluble drug, a gel-forming polymer, and a low-melting lipophilic substance.

3. The sustained-release preparation according to claim 2,
wherein the melt granulation is fluidized bed melt granulation.

4. The sustained-release preparation according to any one of claims 1 to 3, wherein the amount of the gel-forming polymer is from 40 to 80% by mass of the total mass of the preparation.

5. The sustained-release preparation according to any one of claims 1 to 4, wherein the amount of the low-melting lipophilic substance is from 2 to 30% by mass of the total mass of the preparation.

6. The sustained-release preparation according to any one of claims 1 to 5, wherein the amount of the water-soluble drug, gel-forming polymer, and low-melting lipophilic substance is from 95.0 to 99.9% by mass of the total mass of the preparation.

7. The sustained-release preparation according to any one of claims 1 to 6, wherein the gel-forming polymer is one or more members selected frommethyl cellulose, hydroxypropyl cellulose, carmellose sodium, povidone, polyvinyl alcohol, and carboxy vinyl polymer.

8. The sustained-release preparation according to any one of claims 1 to 7, wherein the gel-forming polymer is hydroxypropyl cellulose.

9. The sustained-release preparation according to any one of claims 1 to 8, wherein the gel-forming polymer has an average particle diameter of 200 µm or less.

10. The sustained-release preparation according to any one of claims 1 to 9, wherein the gel-forming polymer has a viscosity of from 1000 to 40000 mPa·s.

11. The sustained-release preparation according to any one of claims 1 to 10, wherein the low-melting lipophilic substance is stearyl alcohol or stearic acid.

12. The sustained-release preparation according to any one of claims 1 to 11, further comprising a lubricant.

13. The sustained-release preparation according to any one of claims 1 to 12, wherein the water-soluble drug has a solubility in a phosphate buffer (pH 6.8) of 300 mg/mL or more.

14. The sustained-release preparation according to any one of claims 1 to 13, wherein the water-soluble drug is metformin or cevimeline hydrochloride or a hydrate thereof.

15. The sustained-release preparation according to any one of claims 1 to 14, wherein the dosage form of the preparation is a tablet.

16. The sustained-release preparation according to claim 15,
wherein the dosage form of the preparation is a press-coated tablet.

17. The sustained-release preparation according to claim 16,
wherein the dosage form of the preparation is a press-coated tablet, and the blending ratio of the water-soluble drug to the gel-forming polymer in the core tablet is from 1:2 to 4:1, and the blending ratio of the water-soluble drug to the gel-forming polymer in the outer shell layer is from 1:2 to 1:6.

18. The sustained-release preparation according to any one of claims 15 to 17, wherein the tablet has a diameter of 10.0 mm or less.

19. The sustained-release preparation according to any one of claims 15 to 18, wherein the tablet has a hardness of from 7.0 to 15.0 kp.

20. The sustained-release preparation according to any one of claims 1 to 19, wherein the preparation is for oral administration.

21. The sustained-release preparation according to any one of claims 1 to 20, wherein, in the dissolution test according to Japanese Pharmacopoeia Dissolution Test Method 2, dissolution rates of the water-soluble drug from the preparation after the lapse of 2 hours, 4 hours, and 6 hours from initiation of the dissolution test are from 5 to 60%, from 10 to 70%, and from 20 to 90%, respectively.

22. The sustained-release preparation according to any one of claims 1 to 21, wherein, in the dissolution test according to Japanese Pharmacopoeia Dissolution Test Method 2, dissolution rates of the water-soluble drug from the preparation after the lapse of 3 hours and 6 hours from initiation of the dissolution test are from 15 to 45% and from 30 to 65%, respectively.

23. A method for producing a sustained-release preparation,
**characterized by** comprising the step of subjecting a water-soluble drug, a gel-forming polymer, and the low-melting lipophilic substance to melt granulation.
